Europäisches Patentamt

European Patent Office

Office européen des brevets

(19)

(11) Veröffentlichungsnummer : **0 041 938**
**B1**

(12) EUROPÄISCHE PATENTSCHRIFT

(45) Veröffentlichungstag der Patentschrift :
28.08.85

(51) Int. Cl.⁴ : **A 61 K 6/04, C 22 C 19/00**

(21) Anmeldenummer : **81890078.9**

(22) Anmeldetag : **14.05.81**

(54) **Dentallegierung.**

(30) Priorität : **22.05.80 AT 2734/80**

(43) Veröffentlichungstag der Anmeldung :
**16.12.81 Patentblatt 81/50**

(45) Bekanntmachung des Hinweises auf die Patenterteilung : **28.08.85 Patentblatt 85/35**

(84) Benannte Vertragsstaaten :
**BE CH DE FR GB IT LI LU NL SE**

(56) Entgegenhaltungen :
DE-A- 707 014
FR-A- 2 189 526
GB-A- 423 562
US-A- 3 808 606
International Metals Review, Tomb 22, Seiten 39-60
1977

(73) Patentinhaber : **VEREINIGTE EDELSTAHLWERKE
AKTIENGESELLSCHAFT (VEW)**
**Elisabethstrasse 12**
**A-1010 Wien (AT)**

(72) Erfinder : **Stierschneider, Hubert**
**Lerchenweg 26**
**A-8600 Bruck a.d. Mur (AT)**
Erfinder : **Kulmburg, Alfred, Dr.**
**Richard-Wagner-Gasse 37**
**A-8605 Kapfenberg (AT)**

(74) Vertreter : **Widtmann, Georg, Dr.**
**Elisabethstrasse 12**
**A-1010 Wien (AT)**

## Beschreibung

Die Erfindung bezieht sich auf die Verwendung einer Kobalt-Chrom-Basis-Legierung für keramisch aufzubrennende Metallgerüste bei der dentalen Prothetik.

In der dentalen Prothetik wird grundsätzlich zwischen den Gußlegierungen für sogenannte Einstück-Gußprothesen und Metallegierungen für die Aufbrennkeramik unterschieden. Bei den letzteren handelt es sich um speziell für diesen Verwendungszweck entwickelte Legierungen, bei denen neben den an sich für Dentalzwecke geforderten Eigenschaften, wie sie etwa in der Beschreibungseinleitung der DE-AS 27 13 755 erwähnt sind, noch eine Reihe weiterer Eigenschaften voll erfüllt sein müssen, nämlich eine ausgezeichnete Haftung mit der keramischen Verblendung, deren Aussehen überdies nicht verschlechtert werden darf, sowie eine gute Übereinstimmung des thermischen Ausdehnungskoeffizienten mit demjenigen der keramischen Massen, damit während der Abkühlung nach dem Brennen Spannungen weitgehend vermieden werden.

Bisher wurden für den genannten Verwendungszweck zwei verschiedene Legierungstypen herangezogen, nämlich einerseits mit Haftoxidbildnern (insbesondere Sn, Zn, Re, In) versetzte Edelmetallegierungen des Typs Platin/Gold ; Palladium/Gold/Silber ; Palladium/Silber. Andererseits wurden wegen des hohen Preises dieser Edelmetallegierungen auch Edelmetall-freie Legierungen entwickelt, die einen wesentlichen Anteil von Nickel enthalten, soferne dieses Element nicht überhaupt die Basis des betreffenden Legierungstyps darstellt. (Siehe dazu etwa die schon erwähnte DE-AS 27 13 755 sowie die CH-PS 548 452).

Erkenntnisse aus der letzten Zeit deuten aber darauf hin, daß der Nickelgehalt dieser Legierungen für allergische Reaktionen bzw. ganz allgemein für eine verminderte Verträglichkeit beim Patienten verantwortlich sein kann, weshalb die vorliegende Erfindung zur Lösung der Aufgabe dient, eine nickelfreie Dentallegierung für den genannten Verwendungszweck vorzuschlagen.

Überraschenderweise wurde nun gefunden, daß es gelingt, die genannte Zielsetzung mit einer in bestimmten Bereichsgrenzen gehaltenen Kobalt-Chrom-Basis-Legierung zu erreichen und demgemäß besteht das Erfindungskennzeichen in der Verwendung einer in Gewichtsprozenten angeführten Legierung aus 0,1 bis 0,25 % C ; 0,1 bis 3,0 % Si ; 0,1 bis 8,0 % Mn ; 25 bis 35 % Cr, vorzugsweise 27 bis 33 % Cr ; 3 bis 8 % Mo, vorzugsweise 4 bis 7 % Mo ; max. 1,0 % Fe ; max. 0,3 % Ni, vorzugsweise max. 0,2 % Ni ; Rest Co für keramisch zu verblendende Metallgerüste bei der dentalen Prothetik.

Mit der vorgeschlagenen Legierung gelingt es, den Ausdehnungskoeffizienten der Legierung ein wenig größer als denjenigen der keramischen Massen zu halten, um so eine geringfügige Druckspannung in der Verblendung sicherzustellen.

Eine abgewandelte Legierungszusammensetzung für den angesprochenen Verwendungszweck ist dadurch gekennzeichnet, daß sie zusätzlich 0,5 bis 5,0 % Ti enthält.

Beispiel

Aus einer Legierung der Zusammensetzung 0,16 % C, 0,5 % Si, 0,5 % Mn, 31,2 % Cr, 5,5 % Mo, 0,7 % Fe, 0,06 % Ni, Rest Co wurden Dentalprothesen abgegossen, die nachfolgend mit keramischen Verblendungen versehen wurden. Es wurden dafür verschiedenartige keramische Zusammensetzungen verwendet, deren Ausdehnungskoeffizient laut den Angaben der Hersteller im Bereich von 12 bis $14 \times 10^{-6} \times K^{-1}$ liegt. Die Ergebnisse waren hinsichtlich aller für diesen Verwendungszweck maßgebenden Kriterien, insbesondere der Gießfähigkeit, des Aussehens nach dem Brand, der Haftung mit dem keramischen Überzug sowie der für diese Legierung ermittelten und nachfolgend angeführten technologischen Kennwerte vollauf befriedigend :

0,2 Dehngrenze ($R_{p0,2}$) 550-700 N/mm$^2$

Zugfestigkeit ($R_m$) 850-1 000 N/mm$^2$

Dehnung (A) 8-12 %

Härte HV 10 : 300-350.

## Patentansprüche

1. Verwendung einer Kobalt-Chrom-Basis-Legierung aus

0,1 bis 0,25 % C ;
0,1 bis 3,0 % Si ;
0,1 bis 8,0 % Mn ;
25 bis 35 % Cr, vorzugsweise 27 bis 33 % Cr ;
3 bis 8 % Mo, vorzugsweise 4 bis 7 % Mo ;
max. 1,0 % Fe ;
max. 0,3 % Ni, vorzugsweise max. 0,2 % Ni ;
Rest Co

für keramisch zu verblendende Metallgerüste bei der dentalen Prothetik.

2. Verwendung der Legierung nach Anspruch 1 mit der Maßgabe, daß sie zusätzlich 0,5 bis 5,0 % Ti enthält, für den in Anspruch 1 genannten Zweck.

## Claims

1. The use of a cobalt-chrome-base alloy of

0.1 to 0.25 % C ;
0.1 to 3.0 % Si ;
0.1 to 8.0 % Mn ;
25 to 35 % Cr, preferably 27 to 33 % Cr ;
3 to 8 % Mo, preferably 4 to 7 % Mo ;
max. 1.0 % Fe ;
max. 0.3 % Ni, preferably max. 0.2 % Ni ;
remainder Co

for metal frames to be faced ceramically in prosthetic dentistry.

2. The use of the alloy according to Claim 1 with the provision that it also contains from 0.5 to 5.0 % Ti for the purpose described in Claim 1.

**Revendications**

1. Utilisation d'un alliage à base de cobalt-chrome ayant pour composition

0,1 à 0,25 % de C ;
0,1 à 3,0 % de Si ;
0,1 à 8 % de Mn ;
25 à 35 % de Cr, de préférence 27 à 33 % de Cr ;
3 à 8 % de Mo, de préférence 4 à 7 % de Mo ;
au maximum 1,0 % de Fe ;
au maximum 0,3 % de Ni, de préférence au maximum 0,2 % de Ni ;
le reste en Co,

pour des carcasses métalliques devant recevoir un revêtement céramique, en prothèse dentaire.

2. Utilisation de l'alliage selon la revendication 1 modifié en ce qu'il contient en plus 0,5 à 5,0 % de Ti pour le but indiqué à la revendication 1.